(19) Europäisches Patentamt
European Patent Office
Office européen des brevets

(11) **EP 4 186 515 A1**

(12) **EUROPEAN PATENT APPLICATION**
published in accordance with Art. 153(4) EPC

(43) Date of publication:
**31.05.2023 Bulletin 2023/22**

(21) Application number: **21845355.3**

(22) Date of filing: **16.07.2021**

(51) International Patent Classification (IPC):
**A61K 31/714** (2006.01)    **A61K 31/201** (2006.01)
**A61P 13/12** (2006.01)    **A61P 43/00** (2006.01)

(52) Cooperative Patent Classification (CPC):
**A61K 31/201; A61K 31/714; A61P 13/12;
A61P 43/00**

(86) International application number:
**PCT/JP2021/026807**

(87) International publication number:
**WO 2022/019235 (27.01.2022 Gazette 2022/04)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.07.2020 JP 2020125095**

(71) Applicants:
• **Niigata University
Niigata 950-2181 (JP)**
• **Denka Company Limited
Tokyo 103-8338 (JP)**

(72) Inventors:
• **SAITO, Akihiko
Niigata-shi, Niigata 951-8510 (JP)**
• **GOTO, Sawako
Niigata-shi, Niigata 951-8510 (JP)**
• **HIRAYAMA, Yoshiaki
Tokyo 103-8338 (JP)**

(74) Representative: **Vossius & Partner
Patentanwälte Rechtsanwälte mbB
Siebertstraße 3
81675 München (DE)**

(54) **UTILIZATION OF VITAMIN B12 IN INHIBITING RENAL DISORDER**

(57) One object of the present invention is to provide an inhibitor for renal injuries induced by an iodinated contrast medium. In this invention, vitamin $B_{12}$ or a pharmaceutically acceptable salt thereof is used.

Fig. 1

EP 4 186 515 A1

**Description**

TECHNICAL FIELD

The present invention relates to utilization of vitamin $B_{12}$ in inhibition of renal injuries. More specifically, this invention relates to an inhibitor for renal injuries induced by iodinated contrast media, the inhibitor comprising vitamin $B_{12}$ or a pharmaceutically acceptable salt thereof. The present invention also relates to a composition for inhibiting the binding between an iodinated contrast medium and transcobalamin 2, the composition comprising vitamin $B_{12}$ or a pharmaceutically acceptable salt thereof as the active component.

BACKGROUND ART

Radiographic contrast techniques are of particularly important significance in clinical applications, including identification of pathological conditions and determination of therapeutic strategies. However, unlike bones, soft tissues are difficult to visualize because attenuation of X-rays is low in soft tissues. It is known that attenuation of X-rays is proportional to the density of a material and to the cube of the atomic number of an element constituting the material. Much interest has been focused on the use of iodine compounds in the development of transvascularly used contrast media. Such contrast media comprising an iodine compound as an active component are called iodinated contrast media.

All materials currently used as water-soluble contrast media have a triiodobenzene ring as a backbone. There are two types of contrast media having this backbone: ionic and nonionic. Ionic contrast media are known to increase osmotic pressure at the same iodine concentration, causing various side effects such as vascular pain, and thus are not approved for intravascular use in Japan.

Nonionic contrast media are developed for the purpose of ensuring enhanced safety through reduction of osmotic pressure. Representative examples of transvascularly used nonionic contrast media include ioxilan, iopromide, iohexol, ioversol, and iomeprol. These contrast media are almost the same in molecular weight and iodine content per unit volume. For this reason, the degrees of X-ray attenuation (contrast effects) of these contrast media are considered nearly comparable. Further, for the purpose of reducing the number of molecules per unit iodine content, dimeric contrast media having two triiodobenzene rings in the molecule, such as iotrolan and iodixanol, are developed, whereby the physical property described as isosmoticity is achieved.

One of known severe side effects of contrast media is contrast nephropathy. Therefore, there is concern about the onset and progression of nephropathy. Further, in some patients with reduced renal function, the use of a contrast medium is in principle contraindicated and thus has to be abandoned.

Possible mechanisms for the onset of contrast-induced renal injuries include decreased renal blood flow and renal medullary hypoxia due to vasoconstriction, and damages caused by active oxygen. Therefore, many clinical studies have been performed in hopes of determining whether N-acetylcysteine or statin having antioxidant activity, or hANP having vasodilatory and renal blood flow enhancing activities, is capable of preventing the onset of contrast-induced nephropathy. However, none of these agents were proved to be effective for such injuries.

The currently recommended method of preventing contrast-induced renal injuries is intravenous administration of isotonic fluids such as normal saline or sodium bicarbonate fluid before and after contrast examination (NPL 1). However, the effectiveness of this preventive method is not supported by a high level of evidence.

PTL 1 describes an inhibitor for renal injuries induced by an iodinated contrast medium. The active component of this inhibitor is cilastatin or a pharmaceutically acceptable salt thereof. Cilastatin inhibits the binding between megalin and an iodinated contrast medium and demonstrates the inhibitory effect.

CITATION LIST

PATENT LITERATURE

PTL 1: International Patent Publication No. WO2019/208777

NON PATENT LITERATURE

NPL 1: Guidelines on the Use of Iodinated Contrast Media in Patients with Kidney Disease 2012, JSN, JRS and JCS Joint Working Group, p.50-79

SUMMARY OF INVENTION

TECHNICAL PROBLEM

An object of the present invention is to provide a novel solution for inhibiting renal injuries induced by an iodinated contrast medium.

SOLUTION TO PROBLEM

The present inventors have conducted intensive studies to achieve the aforementioned object and as a result found that vitamin $B_{12}$ is effective for inhibition of renal injuries induced by iodinated contrast media. The present inventors also found that vitamin $B_{12}$ inhibits the binding between an iodinated contrast medium and transcobalamin 2.
The present invention includes, but is not limited to, the following embodiments.

[1] An inhibitor for renal injuries induced by an iodinated contrast medium, the inhibitor comprising vitamin $B_{12}$ or a pharmaceutically acceptable salt thereof as an active component.
[2] The inhibitor as set forth in [1], wherein the vitamin $B_{12}$ or a pharmaceutically acceptable salt thereof is selected from the group consisting of cyanocobalamin, hydroxocobalamin, methylcobalamin, adenosylcobalamin, and pharmaceutically acceptable salts thereof.
[3] The inhibitor as set forth in [1] or [2], wherein the iodinated contrast medium is selected from the group consisting of compounds having a chemical structure containing one or more 2,4,6-triiodophenyl groups optionally substituted by a substituent at 3 position and/or 5 position, and pharmaceutically acceptable salts thereof.
[4] The inhibitor as set forth in any one of [1] to [3], further comprising cilastatin or a pharmaceutically acceptable salt thereof.
[5] The inhibitor as set forth in any one of [1] to [4], wherein the inhibitor is in an injectable form.
[6] A composition for inhibiting a binding between an iodinated contrast medium and transcobalamin 2, the composition comprising vitamin $B_{12}$ or a pharmaceutically acceptable salt thereof as an active component.

ADVANTAGEOUS EFFECTS OF INVENTION

The present invention can inhibit renal injuries induced by an iodinated contrast medium.
In connection with the present invention, it was found that an iodinated contrast medium binds to transcobalamin 2, and the produced conjugate binds to megalin. It is then considered that the iodinated contrast medium is intracellularly absorbed through megalin and causes renal injuries. It was found that vitamin $B_{12}$ inhibits such a contrast medium from binding to transcobalamin 2. Therefore, it is considered that vitamin $B_{12}$ inhibits renal injuries induced by the iodinated contrast medium through such an inhibition.
It was also found that when vitamin $B_{12}$ and cilastatin are used in combination, renal injuries induced by an iodinated contrast medium can be more effectively inhibited.
As used herein in connection with renal injuries, the term "inhibit (inhibiting or inhibition)" refers to, for example, complete prevention of the onset of a symptom caused by an iodinated contrast medium, reduction of a symptom as compared to when using an iodinated contrast medium alone, or enabling use of an iodinated contrast medium in a subject in which use of an iodinated contrast medium has been abandoned due to a previously existing symptom. The term "reduction" as referred to above includes decreasing the severity of the symptom, and complete elimination of the symptom. Herein, complete prevention of the onset of a disease symptom, or reduction of a symptom as compared to when using a contrast medium alone, is referred to as "prevention", and also included in the scope of the term "inhibition". Further, a medicament for "inhibiting" renal injuries may also be referred to as an "inhibitor".

BRIEF DESCRIPTION OF DRAWINGS

[Fig. 1] Fig. 1 shows the ratios of iodine content to nitrogen content (I/N ratios) in the kidney sections as measured by electron probe micro analyzer (EMPA).
[Fig. 2] Fig. 2 shows immunohistochemistry images of kidney injury molecule-1 (KIM-1) in the kidney cortex.
[Fig. 3] Fig. 3 shows images of periodic acid-Schiff (PAS) stain in the kidney cortex.
[Fig. 4] Fig. 4 shows quartz crystal microbalance (QCM) analysis of the interaction between transcobalamin 2 (TCN2) and megalin.
[Fig. 5] Fig. 5 shows concentration-dependent increase in TCN2 bands obtained by performing the Western blotting with anti-TCN2 antibodies .

[Fig. 6] Fig. 6 shows concentration-dependent decrease in TCN2 bands by competition with cyanocobalamin.

[Fig. 7] Fig. 7 shows concentration-dependent decrease in TCN2 bands by competition with cyanocobalamin.

[Fig. 8] Fig. 8 shows concentration-dependent decrease in TCN2 bands by competition with cyanocobalamin.

[Fig. 9] Fig. 9 shows concentration-dependent decrease in TCN2 bands by competition with methyl cobalamin.

[Fig. 10] Fig. 10 shows concentration-dependent decrease in TCN2 bands by competition with iomeprol, cilastatin and hydroxocobalamin.

[Fig. 11] Fig. 11 shows the I/N ratios in the kidney sections of vitamin $B_{12}$+cilastatin treated mice and normal saline-treated control mice as measured by EMPA.

[Fig. 12] Fig. 12 shows I/N ratios in the kidney sections of cilastatin-treated mice and normal saline-treated control mice as measured by EMPA.

[Fig. 13] Fig. 13 shows I/N ratios in the kidney sections of cilastatin-, cobalamin- and cobalamin+cilastatin-treated mice adjusted by I/N ratios of normal saline-treated control mice as measured by EMPA.

[Fig. 14] Fig. 14 shows urinary KIM-1 levels of each group.

[Fig. 15] Fig. 15 shows mouse mortality rates after administration of iodinated contrast medium.

DESCRIPTION OF EMBODIMENTS

(Vitamin $B_{12}$)

Vitamin $B_{12}$ refers to cobalamin in a broad sense, and cyanocobalamin in a narrow sense. Vitamin $B_{12}$ used in the present invention is cobalamin in the broad sense.

In the present invention, vitamin $B_{12}$ or a pharmaceutically acceptable salt thereof is used. For the sake of confirmation, when vitamin $B_{12}$ produces a hydrate, use of the hydrate is also included within the scope of this invention. This acknowledgement in respect of a hydrate is also applicable to other active components to be described later.

The type of vitamin $B_{12}$ or a pharmaceutically acceptable salt thereof is not particularly limited, but is preferably selected from the group consisting of cyanocobalamin, hydroxocobalamin, methylcobalamin, adenosylcobalamin, and pharmaceutically acceptable salts thereof. A particularly preferred vitamin $B_{12}$ or a pharmaceutically acceptable salt thereof is selected from the group consisting of cyanocobalamin, hydroxocobalamin, methylcobalamin, and pharmaceutically acceptable salts thereof.

Examples of a pharmaceutically acceptable salt of vitamin $B_{12}$ include alkali metal salts, such as lithium salt, sodium salt and potassium salt; alkali earth metal salts, such as magnesium salt and calcium salt; zinc salt and aluminum salt; organic amine salts, such as choline salt, ethanolamine salt, trimethylamine salt, triethylamine salt, dicyclohexylamine salt, dibenzylamine salt, phenethylbenzylamine salt, procaine salt, morpholine salt, pyridine salt, piperidine salt, piperadine salt and N-ethylpiperidine salt; ammonium salt; basic amino acid salts, such as lysine salt and arginine salt; inorganic acid salts, such as sulfate, hydrochloride, hydrobromide, nitrate, phosphate, and perchlorate; organic acid salts, such as formate, acetate, trifluoroacetate, oxalate, lactate, maleate, fumarate, tartrate, citrate, and ascorbate; and sulfonates, such as methanesulfonate, isetionate, benzenesulfonate, p-toluenesulfonate, and camphorsulfonate. The inhibitor of the present invention may contain two or more types of vitamin $B_{12}$ salts. Herein, it is stated for confirmation that the scope of pharmaceutically acceptable salts of all active components also includes hydrates of the salts.

As vitamin $B_{12}$ or a pharmaceutically acceptable salt thereof, use can be made of, for example, a commercially available product, or a product produced and obtained by a *per se* known method or by a method pursuant to a known method.

Vitamin $B_{12}$ or a pharmaceutically acceptable salt thereof is capable of inhibiting indirect binding of an iodinated contrast medium to megalin.

(Iodinated contrast medium)

In the present invention, renal injuries induced by iodinated contrast media are inhibited by vitamin $B_{12}$ or a pharmaceutically acceptable salt thereof. The iodinated contrast medium is preferably selected from compounds having a chemical structure containing one or more (preferably one, two or three, more preferably one or two, still more preferably one) 2,4,6-triiodophenyl groups optionally substituted by a substituent at 3 position and/or 5 position, and pharmaceutically acceptable salts thereof. The iodinated contrast medium is preferably nonionic. As referred to above, the nonionic contrast medium refers to a contrast medium having no ionic functional group. A typical ionic functional group is a carboxyl group. The iodinated contrast medium is preferably selected from the group consisting of ioxilan, iopromide, iohexol, ioversol, iomeprol, iotrolan, iodixanol, ioxaglic acid, iotroxic acid, amidotrizoic acid, iotalamic acid, and pharmaceutically acceptable salts thereof, more preferably selected from the group consisting of iomeprol, ioversol, iodixanol, iopromide, iohexol, and pharmaceutically acceptable salts thereof.

Examples of pharmaceutically acceptable salts of the aforementioned compounds include the following: when the aforementioned compounds have a carboxyl group, base addition salts formed at the carboxyl group; and when the aforementioned compounds have a basic nitrogen atom, such as an amino group, an imino group, or a basic heterocyclic group, acid addition salts formed at the basic nitrogen atom.

Examples of the base addition salts include alkali metal salts, such as lithium salt, sodium salt and potassium salt; alkali earth metal salts, such as calcium salt and magnesium salt; zinc salt and aluminum salt; ammonium salt; and organic amine salts, such as meglumine salt, choline salt, ethanolamine salt, trimethylamine salt, triethylamine salt, dicyclohexylamine salt, diethanolamine salt, triethanolamine salt, morpholine salt, pyridine salt, piperidine salt, piperadine salt, procaine salt, and N,N'-dibenzylethylenediamine salt.

Examples of the acid addition salts include inorganic acid salts, such as sulfate, hydrochloride, hydrobromide, nitrate, phosphate, and perchlorate; organic acid salts, such as formate, acetate, trifluoroacetate, oxalate, lactate, maleate, fumarate, tartrate, citrate, and ascorbate; and sulfonates, such as methanesulfonate, isetionate, benzenesulfonate, p-toluenesulfonate, and camphorsulfonate.

The inhibitor of the present invention may contain two or more types of contrast medium salts.

As iodinated contrast medium, use can be made of, for example, a commercially available product, or a product produced and obtained by a *per se* known method or by a method pursuant to a known method.

(Inhibitor)

In one aspect, the present invention is directed to an inhibitor for renal injuries induced by an iodinated contrast medium, the inhibitor comprising vitamin $B_{12}$ or a pharmaceutically acceptable salt thereof as an active component. Specific examples of iodinated contrast media, and examples of a preferred iodinated contrast medium are as mentioned above.

Renal injuries can be induced by an iodinated contrast medium through the mediation of megalin. Since the main site of expression of megalin in the body is renal proximal tubular epithelial cells (mainly luminal plasmalemma), vitamin $B_{12}$ or a pharmaceutically acceptable salt thereof is useful for the inhibition (i.e., as an inhibitor) of renal proximal tubular epithelial cell injury induced by an iodinated contrast medium through the mediation of megalin, and renal injuries derived therefrom.

Examples of renal injuries induced by iodinated contrast media include contrast-induced) nephropathy, contrast(-induced) renal injury, contrast(-induced) nephritis, contrast-induced) renal failure, contrast(-induced) renal disease, drug(-induced) nephropathy, drug(-induced) renal injury, drug(-induced) nephritis, drug(-induced) renal failure, drug(-induced) renal disease, acute nephropathy, acute renal injury, acute nephritis, acute renal failure, acute renal disease, chronic nephropathy, chronic renal injury, chronic nephritis, chronic renal failure, chronic renal disease, tubular nephropathy, tubular renal injury, tubular nephritis, tubular renal failure, tubular renal disease, tubulointerstitial nephropathy, tubulointerstitial renal injury, tubulointerstitial nephritis, tubulointerstitial renal failure, tubulointerstitial renal disease, obstructive nephropathy, obstructive renal injury, obstructive nephritis, obstructive renal failure, obstructive renal disease, acute nephritic syndrome, rapidly progressive nephritic syndrome, chronic nephritic syndrome, nephrotic syndrome, renal vasospasm, and acute tubular necrosis.

(Dose of active component)

The inhibitor of the present invention contains vitamin $B_{12}$ or a pharmaceutically acceptable salt thereof in an effective amount for inhibiting renal injuries induced by an iodinated contrast medium. In the case of renal injuries, an exemplary daily dose of vitamin $B_{12}$ or a salt thereof in an adult is from 0.01 to 100 mg, or from 0.1 to 10 mg. To achieve a dose in such a range, the inventive inhibitor can be administered once or in divided doses. The inhibitor may also be administered using an intermittent dosing regimen, such as alternate-day or every three day regimen.

The iodinated contrast medium can be used in combination with vitamin $B_{12}$ or a pharmaceutically acceptable salt thereof. In such a case, the course of therapy with an iodinated contrast medium to be combined is not particularly limited, and can be determined by those skilled in the art depending on the need by reference to known literatures or the like. For example, the package insert of "Iomeron®" produced by Bracco-Eisai Co., Ltd., which is a contrast medium product whose generic name is "iomeprol", states as follows regarding its usage.

[Table 1]

| Single doses for adults (These doses can be increased or decreased as appropriate depending on the age, body weight, symptom and purpose.) | | | | |
|---|---|---|---|---|
| Type of imaging | | Dosage regimen/Iodine content in injectable formulation | | |
| | | 300 mg/mL | 350 mg/mL | 400 mg/mL |
| Cerebral blood vessel angiography | | 5-15 mL | - | - |
| Angiocardiography | Intracardiac angiography | - | 20-50 mL | 20-40 mL |
| | Coronary arterial angiography | - | 3-10 mL | 3-8 mL |
| Thoracic angiography | | 5-50 mL | 5-50 mL | 5-50 mL |
| Abdominal angiography | | 5-60 mL | 5-60 mL | 5-60 mL |
| Extremities angiography | | 10-80 mL | 10-80 mL | - |
| Digital radiography | Venous angiography | 10-50 mL | 10-50 mL | - |
| | Arterial angiography | 3-40 mL | 3-40 mL | - |
| Computer-assisted tomography (CT) | | 40-100 mL | 40-100 mL | - |
| Intravenous urography | | 40-100 mL | 30-100 mL | 50 mL |
| The total dose of multiple treatments is up to 250 mL.<br>For dynamic computer-assisted tomography of the liver region, the maximum dose based on weight is 135 mL (350 mg/mL formulation). | | | | |

(Cilastatin)

The inhibitor of the present invention may further contain another active component. To be specific, the inhibitor of the present invention may contain cilastatin or a pharmaceutically acceptable salt thereof.

Cilastatin refers to (Z)-7-[[(R)-2-Amino-2-carboxyethyl]thio]-2-[[[(S)-2,2-dimethylcyclopropyl]carbonyl]amino]-2-heptenoic acid.

Examples of a pharmaceutically acceptable salt of cilastatin include alkali metal salts, such as lithium salt, sodium salt and potassium salt; alkali earth metal salts, such as magnesium salt and calcium salt; zinc salt and aluminum salt; organic amine salts, such as choline salt, ethanolamine salt, trimethylamine salt, triethylamine salt, dicyclohexylamine salt, dibenzylamine salt, phenethylbenzylamine salt, procaine salt, morpholine salt, pyridine salt, piperidine salt, piperadine salt and N-ethylpiperidine salt; ammonium salt; basic amino acid salts, such as lysine salt and arginine salt. The inhibitor of the present invention may contain two or more cilastatin salts. A particularly preferred salt is cilastatin sodium.

When the inhibitor of the present invention contains cilastatin or a pharmaceutically acceptable salt thereof, the composition contains cilastatin or a pharmaceutically acceptable salt thereof in an effective amount for inhibiting renal injuries induced by an iodinated contrast medium. In the case of renal injuries, an exemplary daily dose of cilastatin or a salt thereof in an adult is from 1.0 to 2.0 g, or from 1.0 to 1.5 g. To achieve a dose in such a range, the inventive inhibitor can be administered once or in divided doses. The inhibitor may also be administered using an intermittent dosing regimen, such as alternate-day or every three-day regimen.

(Transcobalamin 2 binding inhibition)

In one aspect, the present invention is directed to a composition for inhibiting the binding between an iodinated contrast medium and transcobalamin 2, the composition comprising vitamin $B_{12}$ or a pharmaceutically acceptable salt thereof as the active component.

In connection with the present invention, the present inventors found that vitamin $B_{12}$ inhibits the binding between an iodinated contrast medium and transcobalamin 2. Transcobalamin 2 is one of transport proteins for vitamin $B_{12}$ (molecular weight of about 45 kDa). It is considered that this inhibitory effect leads to inhibition of renal injuries induced by an iodinated contrast medium.

The composition of the present invention contains vitamin $B_{12}$ or a pharmaceutically acceptable salt thereof in an effective amount. The effective amount can be determined by reference to the doses and the like as mentioned above in connection with the inhibitor of the present invention.

(Dosage form)

The form of the inhibitor and the composition of the present invention is not particularly limited, and the inhibitor and the composition of this invention can be in the form of, for example, a solid formulation such as powder, granule, capsule, tablet or chewable tablet, a liquid formulation such as solution or syrup, or an injectable, or a spray. A preferred form is an injectable.

(Other components)

The inhibitor and the composition of the present invention may contain a pharmaceutically acceptable carrier when required for pharmaceutical purposes. Examples of such a carrier include excipient and solvent. Examples of additional components that may be contained in the inhibitor of this invention include binder, pH adjustor, disintegrant, chelator, solubilizer, suspending agent, emulsifier, isotonizer, stabilizer, soothing agent, antiseptic, antioxidant, lubricant, corrigent, and colorant.

Examples of excipients include organic excipients, such as sugars like lactose, glucose and D-mannitol, starches, and celluloses like crystalline cellulose; and inorganic excipients, such as dicalcium phosphate, calcium carbonate and kaolin. Examples of solvents include purified water and normal saline. Examples of binders include pregelatinized starch, gelatin, gum Arabic, methylcellulose, carboxymethylcellulose, carboxymethylcellulose sodium, crystalline cellulose, D-mannitol, trehalose, hydroxypropylcellulose, hydroxypropylmethylcellulose, polyvinylpyrrolidone, and polyvinyl alcohol. Examples of pH adjustors include hydrochloric acid and sodium hydroxide. Examples of disintegrants include low-substituted hydroxypropylcellulose, chemically modified celluloses and starches, and alginic acid. Examples of chelators include calcium disodium edetate hydrate and calcium sodium edetate hydrate. Examples of solubilizers include polyethylene glycol, propylene glycol, trehalose, benzyl benzoate, ethanol, sodium carbonate, sodium citrate, sodium salicylate and sodium acetate. Examples of suspending agents or emulsifiers include sodium lauryl sulfate, gum Arabic, gelatin, lecithin, glyceryl monostearate, polyvinyl alcohol, polyvinylpyrrolidone, celluloses like carboxymethylcellulose sodium, polysorbates, and polyoxyethylene hydrogenated castor oil. Examples of isotonizers include sodium chloride, potassium chloride, sugars, glycerin, and urea. Examples of stabilizers include polyethylene glycol, sodium dextran sulfate, and other amino acids. Examples of soothing agents include glucose, calcium gluconate and procaine hydrochloride. Examples of antiseptics include p-hydroxybenzoic esters, chlorobutanol, benzyl alcohol, phenethyl alcohol, dehydroacetic acid, and sorbic acid. Examples of antioxidants include sulfite and ascorbic acid.

(Method and use)

In another aspect, the inhibitor of the present invention is directed to use of vitamin $B_{12}$ or a pharmaceutically acceptable salt thereof in the inhibition of renal injuries induced by an iodinated contrast medium. Specific examples and preferred examples of vitamin $B_{12}$, iodinated contrast media, salts thereof, and renal injuries are as mentioned above. Said use may be further combined with administration of an effective amount of an iodinated contrast medium. The iodinated contrast medium can be administered simultaneously with, separately from, or at a time interval from, vitamin $B_{12}$ or a pharmaceutically acceptable salt thereof. For example, the iodinated contrast medium may be administered simultaneously with, prior to, or after administration of vitamin $B_{12}$ or a pharmaceutically acceptable salt thereof. Alternatively, two or more of these administration timings may be adopted in combination.

In another aspect, the inhibitor of the present invention is directed to a method for inhibiting renal injuries induced by an iodinated contrast medium, the method comprising administering an effective amount of vitamin $B_{12}$ or a pharmaceutically acceptable salt thereof to a subject in need thereof. Specific examples and preferred examples of vitamin $B_{12}$, iodinated contrast media, salts thereof, and renal injuries are as mentioned above. Said method may further comprise administering to the subject an effective amount of an iodinated contrast medium. The iodinated contrast medium can be administered simultaneously with, separately from, or at a time interval from, vitamin $B_{12}$ or a pharmaceutically acceptable salt thereof. For example, the iodinated contrast medium may be administered simultaneously with, prior to, or after administration of vitamin $B_{12}$ or a pharmaceutically acceptable salt thereof. Alternatively, two or more of these administration timings may be adopted in combination.

In the case of combined administration as mentioned above, individual components or agents can be administered as separate formulations or as a single formulation.

The effective amount of vitamin $B_{12}$ or a pharmaceutically acceptable salt thereof and the effective amount of an iodinated contrast medium can be determined by reference to the doses and the like as mentioned above in connection with the inhibitor of the present invention.

As referred to herein, the "subject in need thereof (*i.e.*, of inhibiting renal injuries induced by an iodinated contrast medium)" refers to a subject having or at risk of having a symptom of a renal injury, and a subject contraindicated for the administration of an iodinated contrast medium because of the presence of said symptom before the administration of a contrast medium. Said subject is preferably a mammal such as human, a domestic animal such as mouse, rat, rabbit, guinea pig, hamster, monkey, sheep, horse, cow, pig, donkey, dog or cat, or other laboratory animal, with a human being particularly preferred.

In another aspect, the composition for inhibiting the binding between an iodinated contrast medium and transcobalamin 2 of the present invention is directed to use of vitamin $B_{12}$ or a pharmaceutically acceptable salt thereof in the method of inhibiting the binding between an iodinated contrast medium and transcobalamin 2. Specific examples and preferred examples of vitamin $B_{12}$, iodinated contrast medium, and salts thereof are as mentioned above in connection with the inhibitor.

In another aspect, the composition for inhibiting the binding between an iodinated contrast medium and transcobalamin 2 of the present invention is directed to a method for inhibiting the binding between an iodinated contrast medium and transcobalamin 2, the method comprising administering an effective amount of vitamin $B_{12}$ or a pharmaceutically acceptable salt thereof to a subject in need thereof. Specific examples and preferred examples of vitamin $B_{12}$, iodinated contrast medium, and salts thereof are as mentioned above in connection with the inhibitor. Said subject is preferably a mammal such as human, a domestic animal such as mouse, rat, rabbit, guinea pig, hamster, monkey, sheep, horse, cow, pig, donkey, dog or cat, or other laboratory animal, with a human being particularly preferred.

The effective amount of vitamin $B_{12}$ or a pharmaceutically acceptable salt thereof can be determined by reference to the doses and the like as mentioned above in connection with the inhibitor of the present invention.

(Numerical range)

For the sake of clarity, the numerical ranges defined herein by lower and upper limit values, like "from 1.0 to 2.0 g", include the lower and upper limit values.

EXAMPLES

Hereunder, the present invention will be described by way of examples, but this invention is not limited to these examples.

Among iodinated contrast media used (hereinafter also referred simply to as "contrast medium"), iomeprol was a Japanese Pharmacopeia iomeprol injectable solution ["Iomeron® 350" (Bracco-Eisai Co., Ltd.)]. The "bulks" of contrast media used were bulk of iohexol (Tokyo Chemical Industry Co., Ltd.; I0903), bulk of iopromide (Sigma-Aldrich; 1344804-400), bulk of iodixanol (The United State Pharmacopecial Convention, Inc.; 1343517), bulk of ioversol (Toronto Research Chemicals Inc.; 1737000), and bulk of iomeprol (BOC Sciences). For cilastatin, cilastatin sodium (Sigma-Aldrich Japan K.K.: C5743) was used.

The animal species used was C57BL/6J mice (male, aged 9-12 weeks, Charles River Laboratories International).

Vitamin $B_{12}$ used were cyanocobalamin (FUJIFILM Wako Pure Chemical Corporation; 224-00344), methylcobalamin (FUJIFILM Wako Pure Chemical Corporation; 1134-14263), and hydroxocobalamin acetate (FUJIFILM Wako Pure Chemical Corporation; 087-05633).

A model of contrast nephropathy can be constructed when an iodinated contrast medium is administered to C57BL/6J mice as described in PTL 1. The model had, as compared to a control to which the contrast medium was not administered, an elevated serum creatinine level, an increased KIM-1 expression level in renal proximal tubular epithelial cells, and an increased vacuoles in renal proximal tubular epithelial cells. PTL 1 describes that cilastatin inhibits megalin-dependent internalization of iodinated contrast media into renal proximal tubular epithelial cells and cilastatin inhibits renal injuries.

(Test Example 1) Inhibitory activity of vitamin $B_{12}$ on internalization of iodinated contrast medium

The present test example demonstrates that vitamin $B_{12}$ inhibits the internalization of an iodinated contrast medium into renal proximal tubular epithelial cells with the use of mice. For the verification, an electron probe microanalyzer (EPMA) was used.

Methodology

C57BL/6J mice were divided into the following groups and administered with the following agents from the tail vein.

a) Contrast medium + vitamin $B_{12}$-treated group: 100 μL of iomeprol + 100 μL of 1 mg/mL of cyanocobalamin solution

b) Contrast medium + normal saline-treated group (control): 100 μL of iomeprol + 100 μL of normal saline

The contrast medium + vitamin $B_{12}$-treated group was administered with 100 μL of iomeprol + 100 μL of a 1 mg/mL of cyanocobalamin solution (dissolved in normal saline) (total amount of 200 μL), and the contrast medium + normal saline-treated group was administered with 100 μL of iomeprol + 100 μL of normal saline (NS), from the tail vein, and 3 hours later from administration, kidneys were extracted. In order to use right kidneys for analysis, 3 mm-thick sections containing a kidney pedicle portion were prepared from the right kidney, embedded in OCT compound, freeze-fixed with liquid nitrogen, and the frozen-fixed sections were stored at -80°C. The stored sections were sliced at a thickness of 4 μm using a cryostat (CM1850, Leica Biosystems), and the obtained slices were attached onto carbon-coated grids for EPMA and freeze-dried overnight. The prepared sections were analyzed by EPMA (EPMA1610; Shimadzu Ltd, Kyoto, Japan) by following the procedure described below to examine whether there is a difference in the internalization of a contrast medium between the contrast medium + vitamin $B_{12}$-treated group and the contrast medium + normal saline-treated group.

Quantification of elements in biological samples by EPMA

EPMA is an electron microprobe (EMP) instrument that analyzes the constituent elements of a substance of interest by irradiating electron beams onto the substance and measuring the wavelengths and intensities of characteristic X-rays unique to the different elements, which are generated by the electron beams. The iodine content in sections can be determined by EMP analysis (Norby, et al., Scanning Microsc, 1990, vol.4, p.651-666). It has been found that iodinated contrast media are not metabolized in the body. The behaviors of iodinated contrast media *per se* can be verified by analyzing iodine content distribution.

The "thin-film quantification" technique can be applied for analysis of biological samples such as kidney tissue sections. Since the intensity of characteristic X-ray generated when a biological sample of interest is irradiated with an electron beam is proportional to the number of atoms present, comparison of characteristic X-ray intensities enables indirect comparison of the number of atoms.

However, in the "thin-film quantification" technique, simple characteristic X-ray intensities vary depending on the fluctuations in the distributions of cellular components and the influence of the thickness of tissue sections, and thus are difficult to compare in a precise and quantitative manner.

It is reported that in the measurement of biological samples by EPMA, since nitrogen (N) present in biological samples constitutes a main component of cell proteins, the distribution of nitrogen corresponds to the distributions of cellular components in a section, and can be used as an internal standard when taking into account the fluctuations in the distributions of cellular components and the thickness of sections (Tanaka, et al., BMJ Open, 2014, vol.4, e004407; and Moriyama, et al., Am J Respir Crit Care Med, 2007, vol.176, p.70-77).

Therefore, by calculating a ratio of characteristic X-ray intensity between iodine (I) and nitrogen (N) (I/N) in a specified area of biological samples, iodine (I) in different samples can be relatively quantified and compared.

[0059] X-ray count ratio

= (X-ray count of iodine/unit area) / (X-ray count of nitrogen/(unit area))

Based on this principle, the sections obtained by the above experiment were analyzed by EPMA, and iodine in a specified area was relatively quantified and compared among the samples.

Results

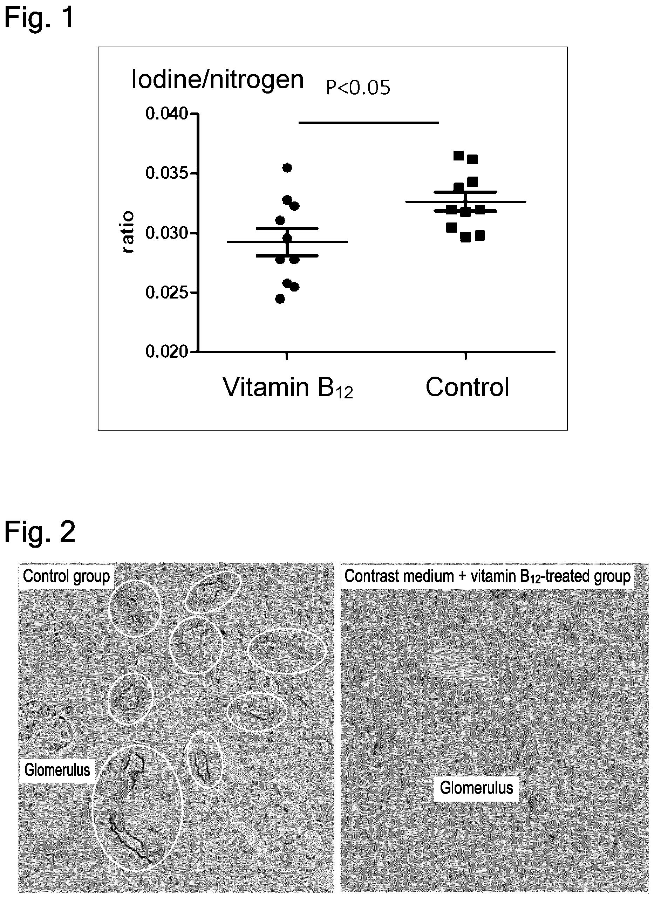

Regions of 1980 μm x 1980 μm containing cortex portions were analyzed, and seven 500 $\mu m^2$ areas were chosen from the cortex areas so as not to overlap with each other. An average was calculated of the I/N X-ray ratios in the seven areas and regarded as an I/N X-ray ratio for one individual. Those I/N X-ray ratios for ten individuals in each of the a) and b) groups were quantified and compared. As a result, the I/N X-ray ratios were significantly (t-test) lower in the a) contrast medium + vitamin $B_{12}$-treated group than in the b) contrast medium + normal saline-treated group (control) (Fig. 1). In other words, it was found that the levels of iodine found in cortex cells in the analyzed slices were lower in the contrast medium + vitamin $B_{12}$-treated group.

From the above results, it was considered that vitamin $B_{12}$ inhibited internalization of contrast media into renal proximal tubular epithelial cells which constituted much of the cortex areas analyzed.

(Test Example 2) Ability of vitamin $B_{12}$ to inhibit renal injuries

In order to confirm the ability of vitamin $B_{12}$ to inhibit renal injuries, C57BL/6J mice were simultaneously administered with an iodinated contrast medium and vitamin $B_{12}$, and the results were analyzed.

Methodology

Left kidneys were extracted from the C57BL/6J mice, and 14 days later, right kidney pedicles were ligated for 30 minutes. Thereafter, reperfusion was carried out. After 24 hours from the start of reperfusion, the C57BL/6J mice were divided into the following groups and administered with the following agents from the tail vein.

a) Contrast medium + vitamin $B_{12}$-treated group: 200 $\mu$L of iomeprol + 100 $\mu$L of 1 mg/mL of cyanocobalamin solution
b) Contrast medium + normal saline-treated group (control): 200 $\mu$L of iomeprol + 100 $\mu$L of normal saline

For each of the groups, 24 hours before anatomizing, urine samples were collected using a metabolic cage, the mice were anatomized after 48 hours from the medication, then urinary kidney injury molecule-1 (KIM-1) was measured. Urinary KIM-1 measurements were conducted by Oriental Yeast Co., Ltd.

Further, right kidneys were extracted and histologically evaluated by immunostaining with KIM-1. To be specific, 3 mm-thick sections containing a kidney pedicle portion were prepared from the right kidneys, fixed with 4% paraformaldehyde phosphate buffer, sliced at a thickness of 4 $\mu$m using a microtome (REM-710; Yamato Kohki Industrial Co., Ltd.), and the obtained thin slices were immunostained with the renal injury marker KIM-1. The immunostaining was performed using VECTASTAIN Elite ABC Kit (Vector Laboratories, Inc.). For the KIM-1 antibody, goat anti-mouse KIM-1 antibody (1:700, AF1817; R&D Systems) was used, and biotin labelled anti-goat IgG (1:200, Vector Laboratories, Inc.) was used as the secondary antibody.

Results

The expression of KIM-1 in tissues was determined exclusively in the outer cortices containing the S1 and S2 segments in which megalin was highly expressed. In the b) contrast medium + normal saline-treated group, KIM-1 was widely expressed, mainly in the S1 and S2 segments (circled portions in Fig. 2). In the contrast medium + vitamin $B_{12}$-treated group, it was confirmed that the expression of KIM-1 was decreased (Fig. 2).

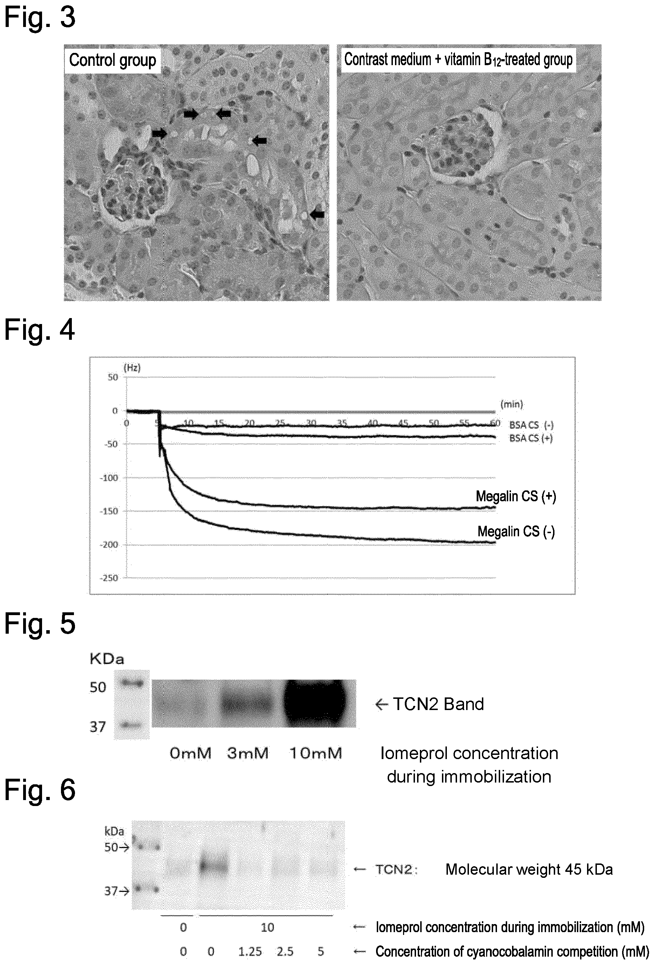

Also, in PAS staining, vacuoles degeneration finding, in other words, injury finding was observed in the b) contrast medium + normal saline-treated group (control) (as indicated by arrows in Fig. 3), whereas the a) contrast medium + vitamin $B_{12}$-treated group did not have such an injury finding.

From the above results, it was found that vitamin $B_{12}$ had an ability to inhibit contrast-induced renal injuries.

(Test Example 3) Binding between transcobalamin 2 and megalin

Inhibitory mechanism of vitamin $B_{12}$ on renal injuries induced by an iodinated contrast medium was verified.

PTL 1 shows that a contrast medium might bind to megalin via a carrier substance and cause renal injuries. Test Example 3 was carried out for the purpose of demonstrating that transcobalamin 2 binds to megalin as such a carrier substance. To be specific, the binding between megalin and transcobalamin 2 was verified by using the quartz crystal microbalance (QCM) method.

Methodology

According to the method of Orlando, *et al*., megalin purified from Sprague-Dawley (SD) rat kidneys was immobilized on quartz crystals. To be specific, with the use of an immobilization kit for AFFINIX® (Initium Inc.) according to the recommended protocol for the kit, a megalin solution was prepared in Buffer A as provided with the kit to a concentration of 62.5 $\mu$g/mL, and placed and immobilized on the quartz crystals mounted in a holder.

After the megalin-immobilized quartz crystals were mounted in the measurement instrument AFFINIX® Q8 (Initium Inc.) and the frequency was confirmed to be stable, recombinant rat transcobalamin 2 proteins (TCN2 LifeSpan Bio Sciences, Inc.) (1.5 $\mu$g) were injected (in total injection volume) to measure frequency over time. More specifically, after the megalin-immobilized quartz crystals were immersed in 200 $\mu$L of buffer and the frequency was confirmed to be stable, transcobalamin 2 was injected and frequency was measured. The same measurement was done on quartz crystals, as a control, on which the same amount of bovine serum albumin (BSA) was immobilized instead of megalin, and the specific binding ability of transcobalamin 2 to megalin was analyzed.

Results

The frequencies decreased only 20 to 30 Hz in the control, whereas the frequencies notably decreased (by about 200 Hz) when transcobalamin 2 was injected (Fig. 4). This demonstrated that transcobalamin 2 bound to megalin.

(Test Example 4) Binding between contrast medium and transcobalamin 2

The binding specificity between transcobalamin 2 and an iodinated contrast medium was verified using contrast medium-immobilized magnetic beads by following the procedures described below.

Methodology

1) Preparation of contrast medium-immobilized beads

The bulk of iodinated contrast medium was dissolved, just before use, in a solvent in which glycidylmethacrylate-coated magnetic beads (FG beads®, particle size: 0.2 $\mu$m, produced by Tamagawa Seiki Co., Ltd.) were dispersed, to initiate chemical bonding between carboxyl groups present on the surface of the magnetic beads and hydroxyl groups in the contrast medium, whereby contrast medium-immobilized magnetic beads were prepared.

The iodinated contrast medium used was the bulk of iomeprol. At feed concentrations of 0 mM, 3 mM and 10 mM, the iodinated contrast medium was immobilized by chemical bonding. The preparation of the iomeprol-immobilized beads was conducted by Tamagawa Seiki Co., Ltd.

2) Preparation of western blotting (WB) samples

5 mg of the iomeprol-immobilized beads prepared at iomeprol feed concentrations of 0 mM, 3 mM and 10 mM in 1) were dispersed and washed in 200 $\mu$L of 100 mM KCL buffer (20 mM HEPES-NaOH (pH 7.9), 100 mM KCl, 1 mM MgClz, 0.2 mM CaCl$_2$, 0.2 mM EDTA, 10% (v/v) glycerol, 0.1% NP-40, 1 mM DTT, 0.2 mM PMSF).

Then, transcobalamin 2 (TCN2: LifeSpan BioSciences, Inc) was dissolved in an aqueous solution of 100 mM KCL to prepare a transcobalamin 2 solution having a concentration of 25 ng/$\mu$L.

The above washed iomeprol-immobilized beads were dispersed in 200 $\mu$L of the transcobalamin 2 solution and incubated at 4°C for 4 hours. After incubation, the iomeprol-immobilized beads were magnetically separated to the bottom part in the solution and the supernatant was removed, and remaining iomeprol-immobilized beads were repeatedly dispersed and washed three times in 200 $\mu$L of 100 mM KCL buffer. Then, 40 $\mu$L of electrophoresis sample buffer (0.25 M Tris-HCl (pH 6.8), 0.02% BPB, 8% SDS, 40% glycerol, 20% 2-mercaptoethanol) mixed with equal volume of distilled water was added to the washed iomeprol-immobilized beads, and the mixture was heated at 98°C for 5 minutes and then the iomeprol-immobilized beads were magnetically separated to collect the supernatant as TCN2-containing samples (western blotting sample).

3) Western blotting analysis using anti-TCN2 antibody

The TCN2-containing samples were subjected to SDS-polyacrylamide electrophoresis under reducing conditions using 4-15% gradient gels (produced by Bio-Rad Laboratories), and the developed samples were transferred onto polyvinylidene fluoride (PVDF) membranes. The membranes were blocked with a buffer containing 5% skim milk and then reacted with polyclonal rabbit anti- TCN2 antibody as a primary antibody (a polyclonal antibody produced by Bioss Inc.) at 2 $\mu$g/mL at room temperature for 2 hours. The membranes were reacted with 0.25 $\mu$g/mL of horseradish peroxidas (HRP)-labeled polyclonal goat anti-rabbit IgG (Dako Ltd.) as a secondary antibody at room temperature for 1 hour. Then, bands were detected using chemiluminescent substrate for western blotting (SuperSignal™ West Femto Maximum Sensitivity Substrate; Thermo Scientific™).

Results

The results are shown in Fig. 5. It was found that, as the concentration of the iodinated contrast medium which was immobilized on the beads increased, the intensity of the band corresponding to TCN2 from heat-eluted samples was increased. Therefore, it was demonstrated that TCN2 specifically bound to the contrast medium immobilized on the beads.

(Test Example 5) Inhibitory effect of vitamin $B_{12}$ on binding between iodinated contrast medium and TCN2

Test Examples 3 and 4 showed that TCN2 functioned as a carrier substance of the iodinated contrast medium and mediated the binding of the contrast medium to megalin. Then, an experiment was carried out to verify the possibility of vitamin $B_{12}$ to inhibit the binding between an iodinated contrast medium and TCN2.

Iodinated contrast media used were the bulk of iomeprol, the bulk of ioversol, the bulk of iodixanol, the bulk of iopromide, and the bulk of iohexol. Evaluations were made using these solid-phased beads. The contrast medium-immobilized beads were prepared by following the procedure described in Test Example 4.

For test chemicals for competing with the binding, vitamin $B_{12}$ (cyanocobalamin, methylcobalamin, or hydroxocobalamin), cilastatin, and iomeprol (iodinated contrast medium) were used. Cilastatin and iomeprol are positive controls of inhibition.

Methodology

1) Samples for competition against the reaction of contrast medium-immobilized beads and TCN2

Different solutions below were prepared using 100 mM KCL buffer (as mentioned in Test Example 4) and reacted at 4°C for 2 hours.

a) Solution containing 25 ng/$\mu$L of TCN2 with no test substance
b) Solutions containing 25 ng/$\mu$L of TCN2 and a test substance

In the b) solutions, the test substances were added at the concentrations shown in Fig. 6 to Fig. 10.

Next, 2 types of 5 mg of the contrast medium-immobilized beads dispersed and washed by the procedure described in Test Example 4 were each added to the above-prepared solutions, and were incubated at 4°C for 4 hours. The solution/beads combinations adopted were as mentioned below.

Solution a) + contrast medium-immobilized beads prepared at 0 mM contrast medium feed concentration (control)
Solution a) + contrast medium-immobilized beads prepared at 10 mM contrast medium feed concentration (no competition)
Solution b) + contrast medium-immobilized beads prepared at 10 mM contrast medium feed concentration (competition with vitamin $B_{12}$, iomeprol, or cilastatin)

Then, these different mixtures were treated by following the procedure described in Test Example 4, and heat-eluted samples (containing TCN2) were prepared from the reaction solutions.

2) WB analysis using antibody

The different heat-eluted samples (containing TCN2) obtained in 1) were subjected to western blotting analysis by following the procedure described in Test Example 4.

Results

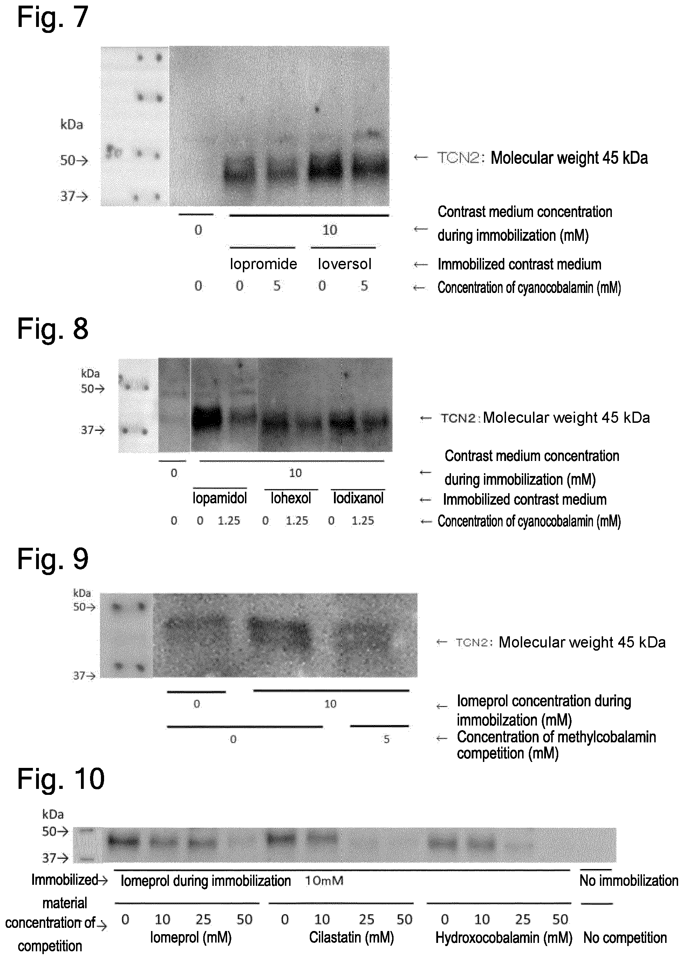

The results are shown in Fig. 6 to Fig. 10. Figs. 6 to 8 show the results of cyanocobalamin, Fig. 9 shows the results of methylcobalamin, and Fig. 10 shows the results of hydroxocobalamin, cilastatin, and the contrast medium (iomeprol) that is not immobilized on the beads.

Since the band of TCN2 was decreased in the coexistence condition of vitamin $B_{12}$, it was considered that the amount of TCN2 bound to the contrast medium-immobilized beads was reduced by vitamin $B_{12}$. Thus, it was found that vitamin $B_{12}$ was capable of inhibiting the binding between each of the iodinated contrast media and TCN2.

(Test Example 6) Inhibitory activity on internalization of iodinated contrast medium by combination use of vitamin $B_{12}$ and cilastatin

PTL 1 describes that cilastatin inhibits renal injuries induced by an iodinated contrast medium. The present test example studied on inhibitory effect on internalization of such a contrast medium into kidneys when vitamin $B_{12}$ and cilastatin were used in combination.

Methodology

Iodine detections by EMPA were compared when vitamin B$_{12}$ (cyanocobalamin) and cilastatin were administered simultaneously.

EMPA analysis was carried out by following the procedure described in Test Example 1. Using C57BL/6J mice (male, aged 9-12 weeks), agents were administered by the following procedures.

Combination group) 100 μL of iomeprol + 50 μL of 2 mg/mL cyanocobalamin solution + 50 μL of 400 mg/kg cilastatin solution

Control group) 100 μL of iomeprol + 100 μL of normal saline

Results

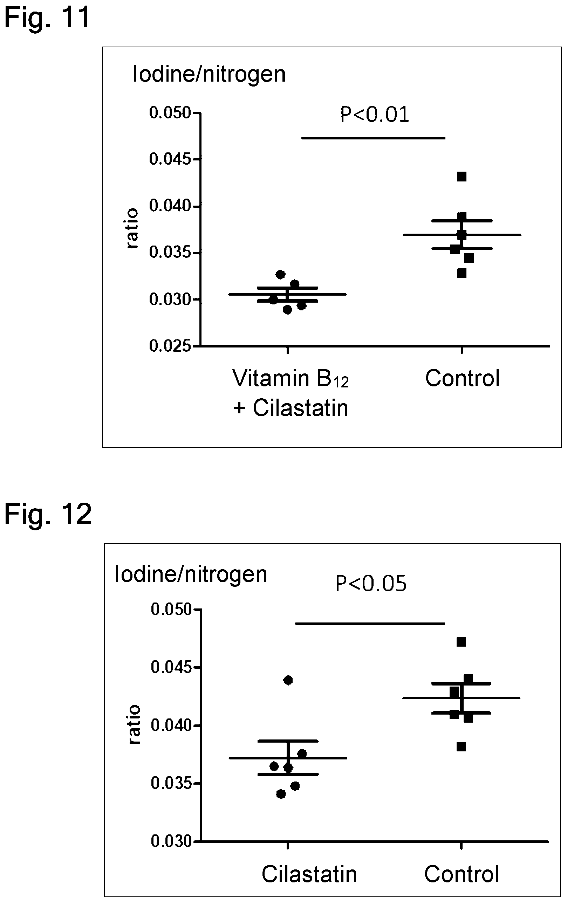

The results are shown in Fig. 11. Detected iodine contents were significantly decreased in the vitamin B$_{12}$ combination group, as compared with the control group (normal saline + iomeprol treated-group). It was found that the combination of vitamin B$_{12}$ and cilastatin inhibited the internalization of an iodinated contrast medium into kidneys.

(Test Example 7) Comparison of inhibitory effects on iodinated contrast medium internalization between cilastatin only and combination use of vitamin B$_{12}$ and cilastatin

In Test Example 6, the internalization of the iodinated contrast medium was inhibited in the vitamin B$_{12}$ + cilastatin combination group. This inhibitory effect was compared with the effect of cilastatin as described in PTL 1.

Methodology-1

The inhibitory effect of cilastatin on internalization of the contrast medium was evaluated by EMPA by following the procedure described in Test example 1. To be specific, using C57BL/6J mice (male, aged 9-12 weeks), agents were administered by the following procedures.

Combination group) 100 μL of iomeprol + 100 μL of 400 mg/kg cilastatin solution

Control group) 100 μL of iomeprol + 100 μL of normal saline

Methodology-2

In order to compare the effects on iodinated contrast medium of a) cyanocobalamin-treated group (Test Example 1), b) cilastatin-treated group (the present Test Example 7/Results-1), and c) cyanocobalamin + cilastatin-treated group (Test Example 6), analysis results of inhibitory effects on the internalization of the contrast medium by EPMA were evaluated based on the I/N ratio of the control normal saline + iomeprol-treated group, to be specific, I/N ratios of test groups a) to c) were each divided by the I/N ratio of the normal saline + iomeprol-treated group to obtain corrected iodine (I) contents (iodine content relative ratio) of the groups a) to c), whereby a) to c) were relatively evaluated.

Results-1

The results are shown in Fig. 12.

Results-2

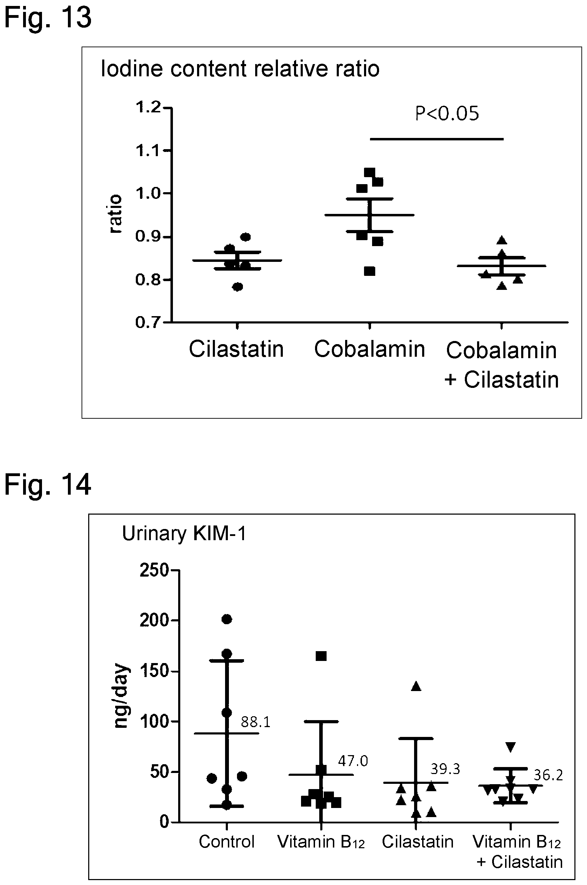

The results are shown in Fig. 13. The c) cyanocobalamin + cilastatin combination group, as compared to a) iodinated contrast medium + cyanocobalamin-treated group (only vitamin B$_{12}$), significantly inhibited the internalization of the contrast medium.

(Test Example 8) Ability of combination use of vitamin B$_{12}$ and cilastatin to inhibit renal injuries

The inhibitory effect of combination use of vitamin B$_{12}$ and cilastatin on iodinated contrast medium-induced renal injuries was studied using renal injury models.

13

Methodology

The procedures used in Test Example 2 were applied *mutatis mutandis* to Test Example 8. Agents were administered by the following conditions.

    a) 200 $\mu$L of iomeprol + 100 $\mu$L of 1 mg/mL cyanocobalamin solution
    b) 200 $\mu$L of iomeprol + 100 $\mu$L of normal saline
    c) 200 $\mu$L of iomeprol + 50 $\mu$L of 2 mg/mL cyanocobalamin solution + 50 $\mu$L of 400 mg/kg cilastatin solution
    d) 200 $\mu$L of iomeprol + 100 $\mu$L of 400 mg/kg cilastatin solution

Results

Fig. 14 shows the measurement results on urinary KIM-1 concentrations 48 hours after contrast administration. It was found that renal injuries were intensely inhibited when cyanocobalamin and cilastatin were used in combination, as compared to when each of vitamin $B_{12}$ (cyanocobalamin) and cilastatin was independently administered.

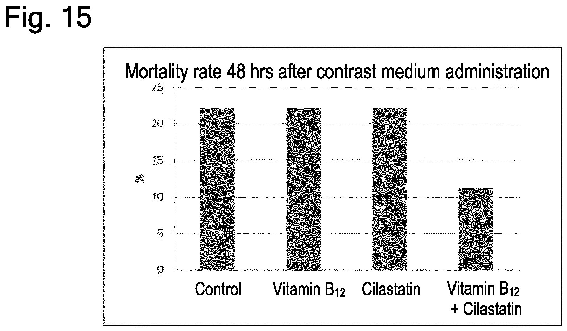

Fig. 15 shows the mortality rate of each group (9 mice per group) 48 hours after contrast medium administration. The combination use of cilastatin and vitamin $B_{12}$ (cyanocobalamin) enabled notable reduction in mortality rate.
It was found, from these findings, that the combination use of vitamin $B_{12}$ and cilastatin provides superior effect.

INDUSTRIAL APPLICABILITY

The present invention is capable of providing an inhibitor for renal injuries induced by an iodinated contrast medium.

**Claims**

1. An inhibitor for renal injuries induced by an iodinated contrast medium, the inhibitor comprising vitamin $B_{12}$ or a pharmaceutically acceptable salt thereof as an active component.

2. The inhibitor according to claim 1, wherein the vitamin $B_{12}$ or a pharmaceutically acceptable salt thereof is selected from the group consisting of cyanocobalamin, hydroxocobalamin, methylcobalamin, adenosylcobalamin, and pharmaceutically acceptable salts thereof.

3. The inhibitor according to claim 1 or 2, wherein the iodinated contrast medium is selected from the group consisting of compounds having a chemical structure containing one or more 2,4,6-triiodophenyl groups optionally substituted by a substituent at 3 position and/or 5 position, and pharmaceutically acceptable salts thereof.

4. The inhibitor according to any one of claims 1 to 3, further comprising cilastatin or a pharmaceutically acceptable salt thereof.

5. The inhibitor according to any one of claims 1 to 4, wherein the inhibitor is in an injectable form.

6. A composition for inhibiting a binding between an iodinated contrast medium and transcobalamin 2, the composition comprising vitamin $B_{12}$ or a pharmaceutically acceptable salt thereof as an active component.

Fig. 1

Fig. 2

## Fig. 3

| Control group | Contrast medium + vitamin B₁₂-treated group |

## Fig. 4

## Fig. 5

← TCN2 Band

0mM   3mM   10mM   Iomeprol concentration
during immobilization

## Fig. 6

← TCN2:   Molecular weight 45 kDa

0              10              ← Iomeprol concentration during immobilization (mM)

0    0   1.25   2.5   5        ← Concentration of cyanocobalamin competition (mM)

## Fig. 7

← TCN2: Molecular weight 45 kDa

← Contrast medium concentration during immobilization (mM)

← Immobilized contrast medium

← Concentration of cyanocobalamin (mM)

## Fig. 8

← TCN2: Molecular weight 45 kDa

← Contrast medium concentration during immobilization (mM)

← Immobilized contrast medium

← Concentration of cyanocobalamin (mM)

## Fig. 9

← TCN2: Molecular weight 45 kDa

← Iomeprol concentration during immobilzation (mM)

← Concentration of methylcobalamin competition (mM)

## Fig. 10

| Immobilized→ material | Iomeprol during immobilization 10mM | | | | | | | | No immobilization |
| concentration of → competition | 0 | 10 | 25 | 50 | 0 | 10 | 25 | 50 | 0 | 10 | 25 | 50 | No competition |
| | Iomeprol (mM) | | | | Cilastatin (mM) | | | | Hydroxocobalamin (mM) | | | | |

Fig. 11

Fig. 12

## Fig. 13

Iodine content relative ratio

P<0.05

ratio: 1.2, 1.1, 1.0, 0.9, 0.8, 0.7

Cilastatin   Cobalamin   Cobalamin + Cilastatin

## Fig. 14

Urinary KIM-1

ng/day: 250, 200, 150, 100, 50, 0

88.1

47.0

39.3

36.2

Control   Vitamin $B_{12}$   Cilastatin   Vitamin $B_{12}$ + Cilastatin

# Fig. 15

Mortality rate 48 hrs after contrast medium administration

**INTERNATIONAL SEARCH REPORT**

| | International application No. |
|---|---|
| | PCT/JP2021/026807 |

**A. CLASSIFICATION OF SUBJECT MATTER**
Int.Cl. A61K31/714(2006.01)i, A61K31/201(2006.01)i, A61P13/12(2006.01)i, A61P43/00(2006.01)i
FI: A61K31/714, A61K31/201, A61P13/12, A61P43/00121
According to International Patent Classification (IPC) or to both national classification and IPC

**B. FIELDS SEARCHED**

Minimum documentation searched (classification system followed by classification symbols)
Int.Cl. A61K31/714, A61K31/201, A61P13/12, A61P43/00

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
JSTPlus/JMEDPlus/JST7580(JDreamIII),
CAplus/REGISTRY/MEDLINE/EMBASE/BIOSIS(STN)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2017-535522 A (FLED HUTCHINSON CANCER RESEARCH CENTER) 30 November 2017 (2017-11-30), claims, paragraphs [0001]-[0003], [0009], [0051], [0076], [0110], [0111], fig. 21 | 1-3, 5-6<br>4 |
| A | 日本医学放射線学会 造影剤安全性委員会, X 線造影剤 製剤別適応一覧表（2020 年 4 月改訂）[online], 公益社団法人 日本医学放射線学会, 01 April 2020, [retrieved on: 04 August 2021], Internet <URL:radiology.jp/content/files/zoei20200401_02.pdf>,「造影剤」の列, (Japan Radiological Society), non-official translation (Japan Radiological Society, Contrast Agent Safety Committee, X-ray contrast agent List of indications by formulation (revised in April 2020), column of "contrast agent") | 1-6 |
| A | WO 2019/208777 A1 (NIIGATA UNIV) 31 October 2019 (2019-10-31), paragraph [0001] | 1-6 |

☒ Further documents are listed in the continuation of Box C.   ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 04 August 2021 | 17 August 2021 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**EP 4 186 515 A1**

**INTERNATIONAL SEARCH REPORT**

International application No.

PCT/JP2021/026807

C (Continuation).   DOCUMENTS CONSIDERED TO BE RELEVANT

| Category* | Citation of document, with indication, where appropriate, of the relevant passages | Relevant to claim No. |
|---|---|---|
| A | 内山幸信, トランスコバラミン欠乏症, Jpn J Electroph, 1994, vol. 38, no. 403, pp. 61-68, table 1, (UCHIYAMA, Yukinobu, Transcobalamin deficiency) | 1-6 |

Form PCT/ISA/210 (second sheet) (January 2015)

22

| INTERNATIONAL SEARCH REPORT | International application No. |
| Information on patent family members | PCT/JP2021/026807 |

| JP 2017-535522 A | 30 November 2017 | WO 2016/053882 A2<br>claims, paragraphs [0003]-[0005],<br>[0011]-[0037], [0079],<br>[0104], [0131], [0132], fig. 21<br>US 2016/0271164 A1<br>KR 10-2017-0057433 A<br>CN 107072190 A |
| WO 2019/208777 A1 | 31 October 2019 | JP 2019-194173 A<br>paragraph [0001] |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019208777 A

**Non-patent literature cited in the description**

- Guidelines on the Use of Iodinated Contrast Media in Patients with Kidney Disease 2012. *JSN, JRS and JCS Joint Working Group,* 50-79
- **NORBY et al.** *Scanning Microsc,* 1990, vol. 4, 651-666
- **TANAKA et al.** *BMJ Open,* 2014, vol. 4, e004407
- **MORIYAMA et al.** *Am J Respir Crit Care Med,* 2007, vol. 176, 70-77